# EUROPEAN PATENT SPECIFICATION

(11) **EP 4 382 065 B1**
(45) Date of publication and mention of the grant of the patent: **31.12.2025**
(21) Application number: 23210220.2
(22) Date of filing: 15.11.2023
(51) Int. Cl.: A61B 34/10, G06N 3/08, G06N 20/00, A61B 34/30

(54) **METHODS FOR TROCHLEAR NOTCH AVOIDANCE**
VERFAHREN ZUR VERMEIDUNG DER TROCHLEAREN KERBE
PROCÉDÉS POUR ÉVITER L'INCISURE TROCHLÉAIRE

(30) Priority: 18.11.2022 US 202263426602 P
(43) Date of publication of application: 12.06.2024
(73) Proprietor: Orthosoft ULC, Montreal , QC H3C 2N6 (CA)
(72) Inventor: SANFORD, Adam H., 55406 Minneapolis (US); MAQUER, Ghislain, 8050 Zurich (CH); HENDERSON, Adam D., 8400 Winterthur (CH)
(74) Representative: Taor, Simon Edward William

(56) References cited:
- US-A1- 2019 272 917
- US-A1- 2022 354 586

## Description

### FIELD

The present invention relates to a computer-implemented method for training a machine learning model for use in predicting bone resection error.

### BACKGROUND

Surgical procedures and instrumentation for knee arthroplasties have been evolving for generations. The instrumentation in particular has evolved to improve accuracy, efficiency, and attempt to prevent common errors. For example, in a partial knee arthroplasty the instruments to resect the distal femur are designed to ensure that the cut is more or less parallel to the proximal tibia. As new technologies, such as robotic surgical systems, are introduced into the field of orthopedics new techniques and instrumentation are being developed that can further improve accuracy, repeatability, flexibility, and efficiency of certain procedures. However, advances in certain areas can come with risks of negative outcomes through use of inappropriate input parameters.

US2019/272917A1 discloses a system and method for pre-operative or intra-operative surgical procedure feedback provide a recommendation to a surgeon. The system may include a robotic surgical device to perform a portion of a surgical procedure on a patient, and a processor to determine a recommendation, based on past surgical information, for the portion of the surgical procedure performed by the robotic surgical device or for a next action to be taken, but the robotic surgical device or a surgeon. The system may include outputting the recommendation by intra-operatively providing the recommendation to a surgeon operating the robotic surgical device.

### OVERVIEW

The invention is set out in the appended set of claims.

As indicated above, robotic surgical platforms are changing the face of orthopedic surgery. For example, robotically assisted total knee arthroplasty has gain general acceptance. In certain robotic surgical techniques cut guides (resection guides) are positioned completely robotically, which eliminates the need for cumbersome and inefficient traditional instrumentation. While the robotic system is tracking the position of the bone and the cut guide in real-time, the lack of standard instrumentation can allow the robot to position a cut guide in a manner that could result in a negative outcome that might not be possible with more traditional instrumentation. In systems that robotically assist with arthroplasty, pre-operative and/or intra-operative planning is done to determine the position and orientation of instrumentation, such as a cut guide. The position and orientation of the cut guide is determined through selection of various resection parameters as well as implant type and sizing, among other things. A computerized planning system can allow a surgeon to have complete control over implant placement within the joint, which along with selection of the implant can control resection parameters. Some planning interfaces will also provide opportunities to tweak certain resection parameters, such as varus-valgus angle or cut depth for example.

Allowing for surgeon control over implant placement and/or resection parameter selection can leave open the opportunity for errors to be introduced into the system. As a robotic surgical system can be more flexible than traditional instrumentation, the system may be able to position a cut guide according to resection parameters (or implant placement selection) that could result in a negative outcome provided that an unfortunate combination of varus-valgus angle and cutting depth was selected. Accordingly, it would be desirable to identify the combinations of resection angle and depth that could potentially lead to negative outcomes before resecting any bone material.

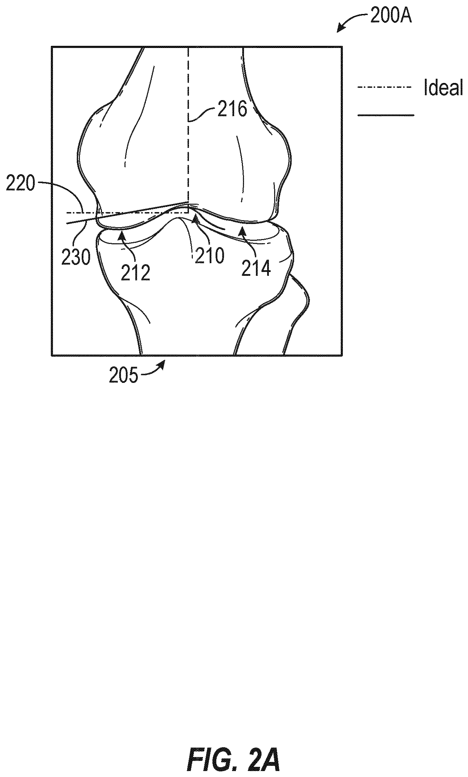

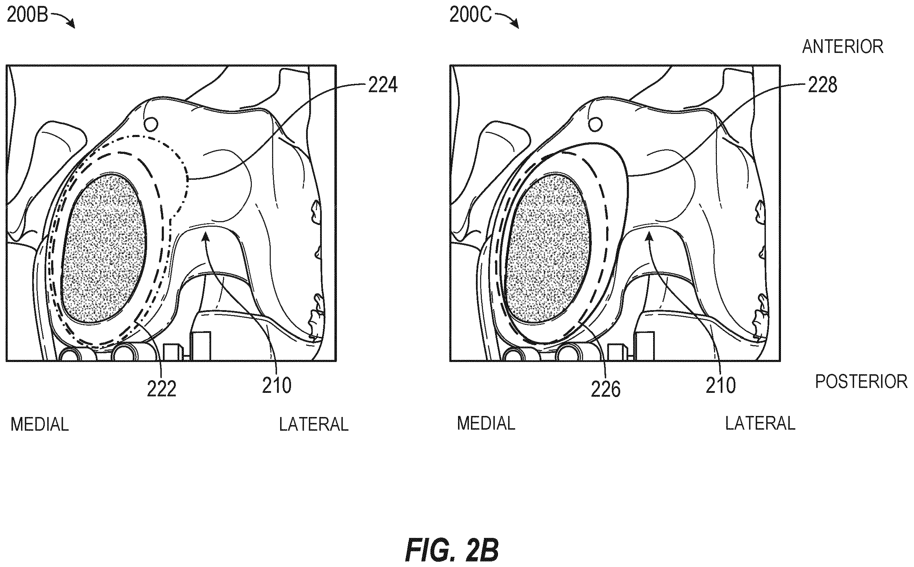

The inventors have developed a number of systems and techniques to check resection parameters prior to implementation by the robotic surgical system to avoid negative outcomes. For example, in a partial knee arthroplasty if the femoral component is placed on the medial condyle in a position that is too valgus (see FIG. 2A, FIG. 2B discussed below), the cut may leave a notch in the lateral condyle running in the anterior-posterior (A-P) direction (more or less where Whiteside's line would be). The system and techniques discussed below provide various automated mechanisms to detect resection parameters during the planning phase (pre-operatively or intra-operatively).

This Overview is intended to provide non-limiting examples of the present subject matter - it is not intended to provide an exclusive or exhaustive explanation. The Detailed Description below is included to provide further information about the present systems, apparatuses, and methods.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a schematic diagram of a robotic surgical system, in accordance with at least one example of the disclosure.
FIG. 2A illustrates example medial condyle resections in accordance with at least one example of this disclosure.
FIG. 2B illustrates various medial condyle resections in an axial view of a distal femur in accordance with at least one example of this disclosure.
FIGS. 3A-3B illustrate example landmark points on the distal femur in accordance with at least one example of this disclosure.
FIG. 4 illustrates an example trochlear depth prediction in accordance with at least one example of this disclosure.
FIG. 5A illustrates a machine learning model training diagram in accordance with at least one example of this disclosure.
FIG. 5B illustrates a machine learning model inference diagram in accordance with at least one example of this disclosure.
FIG. 6 is a flowchart illustrating a technique for predicting bone resection issues in accordance with at least one example of this disclosure.
FIG. 7 is a flowchart illustrating a technique for generating a trochlear notch warning in accordance with at least one example of this disclosure.
FIG. 8 is a flowchart illustrating a technique for training a machine learning model to detect trochlear notching in accordance with at least one example of this disclosure.
FIG. 9 illustrates a block diagram of an example machine upon which any one or more of the techniques discussed herein may perform in accordance with at least one example of this disclosure.

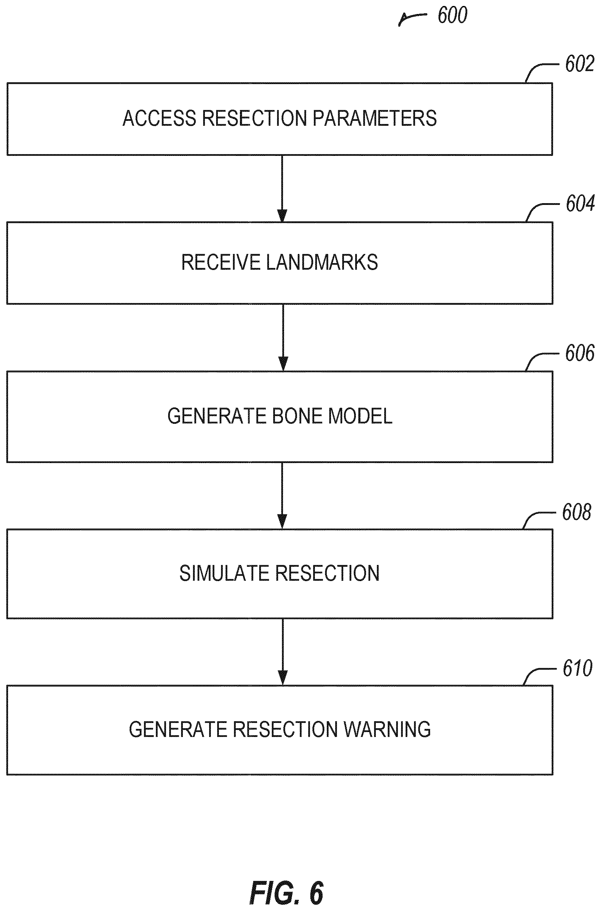

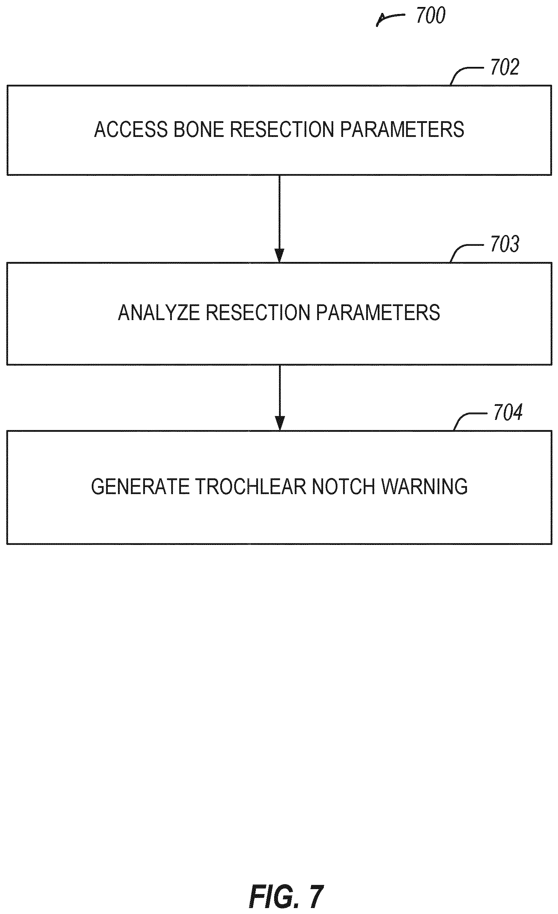

The flow charts of Figures 6 and 7 do not include all steps of the claimed method.

### DETAILED DESCRIPTION

The following discusses subject matter related to techniques for detecting potential issues with planning parameters for robotically assisted arthroplasty procedures. The systems and techniques are discussed below primarily in reference to a robotically assisted partial knee arthroplasty procedure, but could easily be adapted to address other robotically assisted or navigated orthopedic procedures. Implementation for different procedures or different target bones would involve obtaining different landmarks and adjusting different resection parameters, and thus the techniques discussed are readily adaptable.

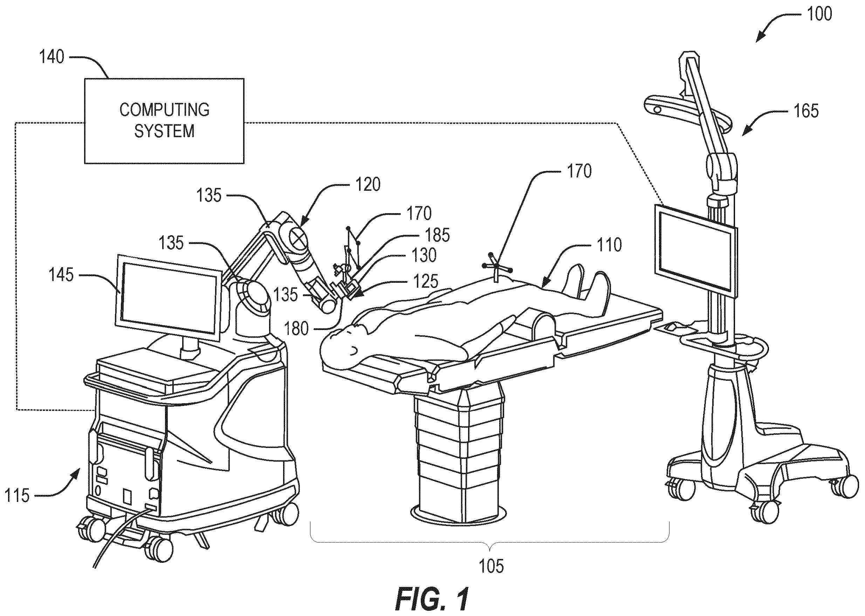

FIG. 1 illustrates a robotic surgical system 100 for operation on a surgical area 105 of a patient 110 in accordance with at least one example of the present disclosure. The surgical area 105 in one example can include a joint and, in another example, is a bone. The surgical area 105 can include any surgical area of the patient 110, including but not limited to the shoulder, hip, knee, and the like. The surgical system 100 also includes a robotic system 115 with one or more robotic arms, such as robotic arm 120. As illustrated, the robotic system 115 will commonly utilize only a single robotic arm. The robotic arm 120 can be a 6 degree-of-freedom (DOF) robot arm, such as the ROSA robot from Medtech, a Zimmer Biomet Inc. company. In some examples, the robotic arm 120 is cooperatively controlled with surgeon input on the end effector or surgical instrument, such as surgical instrument 125 (also referred to herein as end effector 125). In other examples, the robotic arm 120 can operate autonomously. In an example, the robotic surgical system 100 can be configured to assist in performing a knee arthroplasty, and more specifically a partial knee arthroplasty. When configured for assisting with a partial knee arthroplasty, the robotic surgical system 100 can position a cut guide relative to the distal femur of the patient to allow for resection of one of the condyles of the distal femur.

Each robotic arm 120 rotates axially and radially and receives a surgical instrument, or end effector, 125 at a distal end 130. The surgical instrument 125 can be any surgical instrument adapted for use by the robotic system 115, such as a cut guide, a drill or drill guide, or another surgical cutting instrument. The surgical instrument 125 is positionable by the robotic arm 120, which includes multiple robotic joints, such as joint 135, that allows the surgical instrument 125 to be positioned at any desired location adjacent or within a given surgical area 105.

The robotic system 115 also includes a computing system 140 that operates the robotic arms 120 and surgical instrument 125. The computing system 140 can include at least a memory, processing unit, and user input devices, as will be described herein. The computing system 140 also includes a human interface device 145 (e.g., user interface) for providing images for a surgeon to be used during surgery. The computing system 140 is illustrated as a separate standalone system, but in some examples the computing system 140 can be integrated into the robotic system 115. The human interface device 145 provides images, including but not limited to three dimensional images of bones, glenoid, joints, and the like. The human interface device 145 can include associated input mechanisms, such as a touch screen, foot pedals, or other input devices compatible with a surgical environment.

The computing system 140 can receive pre-operative medical images. These images are received in any manner and the images include but are not limited to computed tomography (CT) scans, magnetic resonance imaging (MRI), two dimensional x-rays, ultrasound, and the like. These images in one example are sent via a server as files attached to an email. In another example the images are stored on an external memory device such as a memory stick and coupled to a USB port of the robotic system to be uploaded into the processing unit. In yet other examples, the images are accessed over a network by the computing system 140 from a remote storage device or service.

After receiving one or more images, the computing system 140 can generate one or more virtual models related to surgical area 105. Specifically, a virtual model of the patient's anatomy can be created by defining anatomical points within the image(s) and/or by fitting a statistical shape model to the image data. The virtual model, along with virtual representations of implants, can be used for calculations related to the desired height, depth, inclination angle, or version angle of an implant, stem, surgical instrument, or the like related to be utilized in the surgical area 105. The virtual model can also be used to determine bone dimensions, implant dimensions, resection positions, cut guide position and orientation, and the like. Any model generated, including three dimensional models, can be displayed on the human interface for reference during a surgery or used by the robotic system 115 to determine motions, actions, and operations of a robotic arm 120 or surgical instrument 125. Known techniques for creating virtual bone models can be utilized, such as those discussed in U.S Patent 9,675,461, titled "Deformable articulating templates" or U.S. Patent 8,884,618, titled "Method of generating a patient-specific bone shell" both by Mohamed Rashwan Mahfouz, as well as other techniques known in the art.

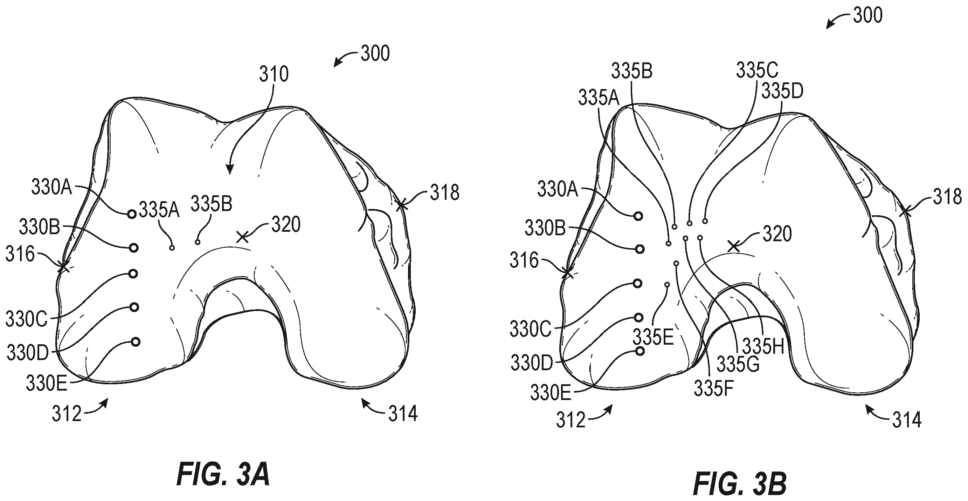

In some examples, the computer system 140 can generate at least partial bone models from landmark digitized on the target anatomy. Various landmarks are discussed below in reference to FIG. 3 that relate to performing resections on a distal femur assisted by the robotic surgical system 100.

The computing system 140 also communicates with a tracking system 165 that can be operated by the computing system 140 as a stand-alone unit. The surgical system 100 can utilize an optical tracking system, such as a Polaris optical tracking system from Northern Digital, Inc. of Waterloo, Ontario, Canada. The tracking system 165 can monitor a plurality of tracking elements, such as tracking elements 170, affixed to object of interest to track locations of multiple objects within the surgical field. The tracking system 165 functions to create a virtual three-dimensional coordinate system within the surgical field for tracking patient anatomy, surgical instruments, or portions of the robotic system 115. The tracking elements 170 can be tracking frames including multiple IR reflective tracking spheres, or similar optically tracked marker devices. In one example, the tracking elements are placed on or adjacent one or more bones of the patient 110. In other examples, the tracking elements 170 can be placed on a robot robotic arm 120, a surgical instrument 125, and/or an implant to accurately track positions within a virtual coordinate system. In each instance the tracking elements provide position data, such as patient position, bone position, joint position, robot robotic arm position, implant position, or the like. The tacking system 165 can also be used to digitize landmark locations intraoperatively. Locating landmarks can be done with a pointer instrument that includes tracking elements, such as tracking elements 170. The pointer instrument is tracked by the tracking system 165 while the physician selects specific points of interest on the target anatomy.

FIG. 2A illustrates example medial condyle resections in accordance with at least one example of this disclosure. As discussed above, one robotically assisted arthroplasty procedure is a partial knee arthroplasty that involves resection of one condyle on the distal femur to install a femoral implant. FIG. 2A illustrates two potential resections, an ideal resection 220 that is roughly parallel to the proximal tibia 205 (and perpendicular to the mechanical axis 216); and a valgus femoral cut 230 that is angled relative to the proximal tibia 205 in a manner that could result in notching the trochlea 210. The distal femur 200A is depicted with the mechanical axis 216 running roughly perpendicular to the tibial plateau of the proximal tibia 205. The ideal resection 220 is also approximately perpendicular to the mechanical axis 216. In certain scenarios, a surgeon may need to adjust the femoral cut to correct an existing deformity. However, the present disclosure includes systems and technique to detect when the adjustment has gone too far and a potential negative outcome is likely.

FIG. 2B illustrates various medial condyle resections in an axial view of a distal femur 200B, 200C in accordance with at least one example of this disclosure. Distal femur 200B illustrates two different resection depths, 6.5mm and 8.5mm. In this example, the 6.5mm resection 222 does not encroach into the trochlea 210 and produces a nice oval resection outline. In contrast, 8.5mm resection 224 illustrates how a deeper resection can encroach into the trochlea 210. The outline of the 8.5mm resection 224 extends laterally into the area of the trochlea 210. The distal femur 200C illustrates resections at the same depth (6.5mm), but with a neutral resection 226 and a valgus resection 228. In this example, the neutral resection 226 includes a profile similar as the 6.5mm resection 222. In contrast, the valgus resection 228 encroaches into the area of the trochlea 210. The valgus resection 228 is angled in a valgus direction relative to the mechanical axis of the femur. Taken to the extreme, both the 8.5mm resection 224 and the valgus resection 228 illustrate resection parameters that could lead to notching of the trochlea (e.g., the inability to complete the femoral resection in a lateral direction). The following techniques provide mechanisms to identify resection parameters let would lead to resections such as the 8.5mm resection 224 and the valgus resection 228, among other potential issues.

FIGS. 3A-3B illustrate example landmark points on the distal femur 300 in accordance with at least one example of this disclosure. The techniques discussed herein for predicting potential negative resection outcomes can utilize a number of different landmarks. FIGS. 3A and 3B illustrate different landmarking techniques that can be used with the techniques discussed in reference to FIGS. 6-8.

FIG. 3A illustrates a variety of different landmarks that can be digitized on a distal femur during an intraoperative procedure. In an example, the navigation system can track a digitizing pointer that can be used by the surgeon to select the various landmarks. In this example, anatomical landmarks can include the medial epicondyle 316, the lateral epicondyle 318 and the canal entry point 320. The distal most point on the medial condyle can be located using condyle landmarks 330A-330E (discussed collectively as condyle landmarks 330). The condyle landmarks 330 can be used to approximate the femoral profile view from the medial lateral direction (j-curve), which can then be used to approximate the distal most point on the condyle. In another example, a computing system can determine from the condyle landmarks 330 a distal most point on the condyle surface (without approximating the j-curve). The distal most point on the condyle surface is a common reference point for pre-operative and intra-operative planning of implant placement. In this example, the landmarks digitized can also include transition landmarks 335A-335B (collectively referred to as transition landmarks 335). The transition landmarks 335 can be used to approximate a two-dimensional (2D) profile of the trochlea 310 by generating a spline between the distal most point on the condylar surface and the canal entry point 320. As discussed further below, some examples can use the approximated profile to predict negative outcomes.

FIG. 3B includes most of the same landmarks as discussed in reference to FIG. 3A but includes a larger number of transition landmarks 335A-335H. In this example, the transition landmarks 335 are used to approximate a three-dimensional (3D) surface of the trochlea area (e.g., trochlea 310).

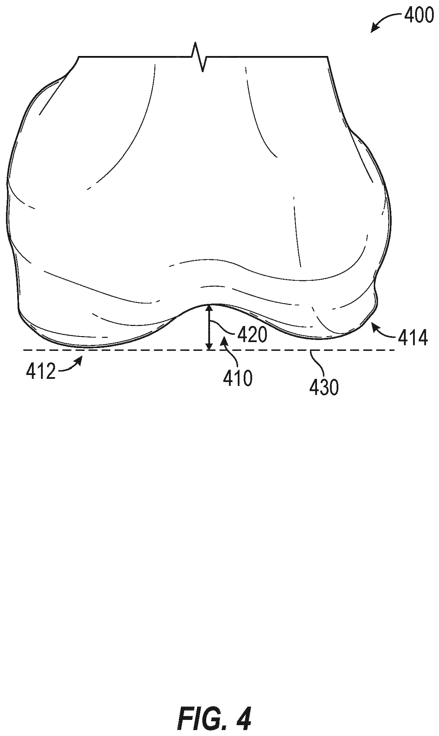

FIG. 4 illustrates an example trochlear depth 420 prediction in accordance with at least one example of this disclosure. In this example, the distal femur 400 includes a medial condyle 412, a lateral condyle 414 and a trochlea 410. The landmarks discussed above in reference to FIGS. 3A and 3B can be used to predict the trochlear depth 420 as measured from a distal plane 430. In this example, the distal plane 430 is perpendicular to the mechanical axis and include the distal most point on the medial condyle 412. FIG. 4 illustrates the distal femur 400 in a two-dimensional profile view in the anterior-posterior direction. In some examples, the trochlear depth can be compared to the resection depth to generate a warning if the depths are too close. The trochlear depth may also be used as an input to training a machine learning model to predict negative resection outcomes.

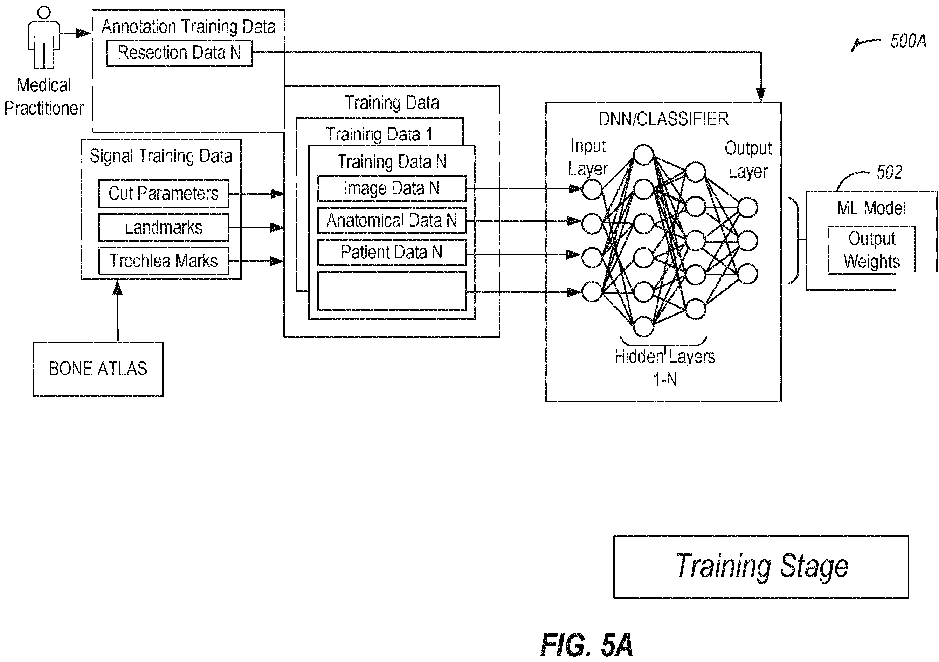

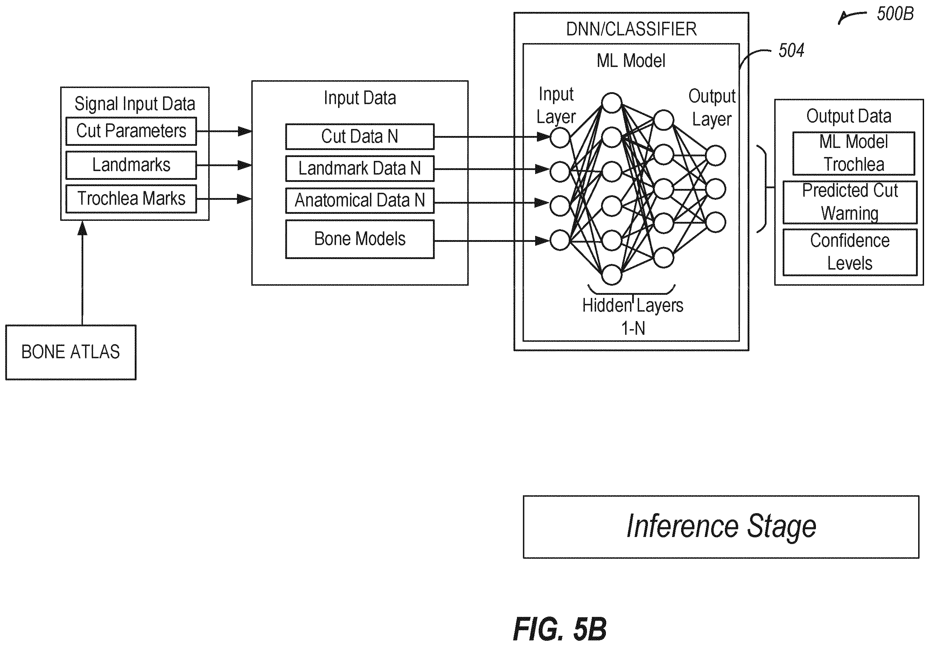

FIG. 5A illustrates a machine learning model training diagram 500A in accordance with at least one example of this disclosure. The diagram 500A illustrates components and inputs for training a model 502 using machine learning. In this example, a machine learning model can be generated that predicts resection issues based on inputs such as resection (cut) parameters and a bone model. In some examples, the bone model can be generated based on digitized landmarks obtained from the distal femur of the patient. In other examples, the bone model is generated from a pre-operative CT or similar imaging scan. As discussed further below, the machine model can be trained by simulating resections on bone models from a statistical bone atlas (which is a database of scans from a wide variety of distal femurs, in this case). The simulated resections can then be labeled to identify issues that the model will then be able to predict in the inference mode (see FIG. 5B).

Machine Learning (ML) is an application that provides computer systems the ability to perform tasks, without explicitly being programmed, by making inferences based on patterns found in the analysis of data. Machine learning explores the study and construction of algorithms, also referred to herein as tools, that may learn from existing data and make predictions about new data. Although examples may be presented with respect to a few machine-learning tools, the principles presented herein may be applied to other machine-learning tools.

The machine-learning (ML) algorithms use data (e.g., action primitives and/or interaction primitives, goal vector, reward, etc.) to find correlations among identified features that affect the outcome. A feature is an individual measurable property of a phenomenon being observed. Example features for the model 502 may include resection data labeling outcomes from simulated resections. The features, which may include and/or be called label data, may be compared to input data, such as resection (cut) parameters, anatomical landmarks, and points transitioning into the trochlea (e.g., transition marks). In an example, the transition marks can be a series of landmarks obtained between a canal entry point and a femoral condyle (which may be represented by a line of landmarks selected to locate the distal most point on femoral condyle). An example of landmarks and transition marks used as inputs is illustrated in FIG. 3 (discussed above). Trochlear depth may be another anatomical input to the training data set.

The concept of a feature is related to that of an explanatory variable used in statistical techniques such as linear regression. Choosing informative, discriminating, and independent features is important for effective operation of ML in pattern recognition, classification, and regression. Features may be of different types, such as numeric features, strings, and graphs.

During training, a ML algorithm analyzes the input data based on identified features and optionally configuration parameters defined for the training (e.g., environmental data, state data, patient data such as demographics and/or comorbidities, etc.). The result of the training is the model 502, which is capable of taking inputs to produce a complex task. In this example, the model 502 will be trained to identify different potential issues that could occur with a given set of resection parameters. In an example, the model 502 will be trained to identify trochlear notching in robotic resection parameters to perform a partial knee arthroplasty resection on one condyle of a distal femur.

In an example, input data may be labeled (e.g., for use as features in a training stage). Labeling may include identifying simulated bone resection outcomes that result in an undesirable outcome (e.g., trochlear notching). Labeled training data may be weighted, and/or may be used to generate different versions of the model 502.

Input training data for the model 502 may include resection parameters, landmarks, transition marks, trochlear depth, and bone models from a bone atlas. In some examples, input training data can also include additional anatomical measurements of the target bone. In the partial knee arthroplasty example, measurements that further describe the distal morphology of the femur could be included, such as medio-lateral and antero-posterior widths.

A neural network (NN) is a computing system based on consideration of biological neural networks of animal brains. Such systems progressively improve performance, which is referred to as learning, to perform tasks, typically without task-specific programming. For example, in image recognition, a neural network may be taught to identify images that contain an object by analyzing example images that have been tagged with a name for the object, and having learned the object and name, may use the analytic results to identify and/or classify the object in untagged images. In FIG. 5A for example, the model 502 may be trained to identify negative resection outcomes, and/or with a percentage likelihood and/or confidence of tissue location. One specific negative resection outcome that may be predicted with model 502 is trochlear notching in a partial knee arthroplasty femoral condyle resection.

A neural network is based on a collection of connected units called neurons, where each connection, called a synapse, between neurons can transmit a unidirectional signal with an activating strength that varies with the strength of the connection. The receiving neuron can activate and propagate a signal to downstream neurons connected to it, typically based on whether the combined incoming signals, which are from potentially many transmitting neurons, are of sufficient strength, where strength is a parameter.

A deep neural network (DNN) is a stacked neural network, which is composed of multiple layers. The layers are composed of nodes, which are locations where computation occurs, loosely patterned on a neuron in the human brain, which fires when it encounters sufficient stimuli. A node combines input from the data with a set of coefficients, and/or weights, that either amplify or dampen that input, which assigns significance to inputs for the task the algorithm is trying to learn. These input-weight products are summed, and the sum is passed through what is called an activation function for a node, to determine whether and to what extent that signal progresses further through the network to affect the ultimate outcome. A DNN uses a cascade of many layers of non-linear processing units for feature extraction and transformation. Each successive layer uses the output from the previous layer as input. Higher-level features are derived from lower-level features to form a hierarchical representation. The layers following the input layer may be convolution layers that produce feature maps that are filtering results of the inputs and are used by the next convolution layer.

The DNN may be a specific type of NN, such as a convolutional neural network (CNN), a recurrent neural network (RNN), a Long Short-Term Memory (LSTM), or the like. Other artificial neural networks may be used in some examples. For example, for resection parameter input data, an identification of negative resection outcome may be generated by the model.

The input data for training the model 502 may include data generated by simulated resections, with labeled data from a medical practitioner. The model 502 may be used in an inference stage (described in further detail below with respect to FIG. 3B) for identifying negative outcomes given a set of input resection parameters and bone shape (model) information.

As shown in FIG. 3A, training data may include signal training data that is comprised of resection (cut) parameters, bone landmarks, and application specific landmarks (e.g., transition marks) which are then applied against bone models from a bone atlas. In some examples, the training data includes annotation training data that is provided by a medical practitioner (e.g., as labeling data). For example, a medical practitioner may annotate each simulated bone resection as acceptable / unacceptable or with a numeric grading scale. This annotated training data may be used to train the model 502.

Based on the signal training data and/or annotation training data, the model 502 may generate output weights corresponding to individual processing nodes that are spread across an input later, an output layer, and one or more hidden layers. The model 502 and trained weights may later be used to infer an indication of problematic resection parameters during a pre-operative planning phase.

In other example, the model 502 may be trained without any input from a medical practitioner, based solely on objective inputs and an objective assessment of resections resulting from the simulated resections based on resection parameters, landmarks, and bone models. The simulated resections can be analyzed for incomplete resections, such as occurs with trochlear notching or other obvious errors in the simulated resection.

FIG. 5B illustrates a machine learning model inference diagram 500B in accordance with at least one example of this disclosure. In the inference diagram 500B, a model 504 (e.g., the model 502 after training, and/or as updated, etc.) may be used to output a prediction, such as a negative resection outcome, or the like. A confidence level and/or weighting may be output as the prediction, or in addition to other predictions discussed above. The machine learning model inference diagram 500B may represent an exemplary computer-based clinical decision support system (CDSS) that is configured to assist in predicting errors in proposed resection parameters, such as parameters that would result in trochlear notching.

As shown in FIG. 5B, the model 504 may receive input data, such as resection parameters and landmarks (standard landmarks and application specification landmarks, such as transition marks). The input data can also include additional anatomical data (e.g, trochlear depth or other bone measurements) and bone models from a statistical atlas of bone models, among other things. The model 504 may generate an output (e.g., an inference) that includes identification of a negative resection outcome, or the like. The model 504 may have a run-time that occurs while a patient is undergoing a robotic arthroplasty procedure or in a pre-operative planning phase prior to the procedure. The model 504 may provide a physician with real-time or near real-time information about the outcome of a proposed set of resection parameters.

FIG. 6 is a flowchart illustrating a technique 600 for predicting bone resection issues in accordance with at least one example of this disclosure. In this example, the technique 600 can include operations such as accessing resection parameters at 602, receiving landmarks at 604, generating a bone model at 606, simulating a resection at 608, and generating a resection warning at 610. In certain examples, the technique 600 can be implemented within a robotic surgical system, such as robotic surgical system 100, assisting a physician in performing an arthroplasty procedure, such as a partial knee replacement. Part of the robotic surgical procedure involves a pre-operative and/or intraoperative planning procedure that includes the physician selecting resection parameters, such as cut depth and various angles (flexion-extension and varus-valgus). In an example, the resection parameters are used by the robotic surgical system to position a cut guide relative to the distal end of the femur. The technique 600 can be used to identify resection parameters that may result in a negative outcome, such as notching the trochlea. Note, techniques 700 and 800 discussed below can also be implemented within the robotic surgical system 100 and can also be implemented within a computerized pre-operative or intra-operative planning system.

In this example, the technique 600 can begin at 602 with a robotic surgical system (such as robotic surgical system 100) accessing a set of resection parameters. In an example, the resection parameters can include resection depth, resection flexion-extension angle, and/or resection varus-valgus angle. Accessing the resection parameters can include receiving input via a user interface, such as touch screen, voice input, or other computer input mechanism. At 604, the technique 600 can continue with the robotic surgical system 100 receiving a plurality of landmarks digitized on the bone to be cut. In an example, the robotic surgical system 100 can include an optical navigation system (e.g., tracking system 165) that can track a digitizing pointer used to select landmarks on the distal femur.

At 606, the technique 600 can continue with a computing system coupled to the robotic surgical system 100 (e.g., computing system 140) generating a bone model. In an example, the bone model can be generated from various landmarks digitized on the target bone. In certain examples, the bone model can approximate a portion of the distal surface of the distal femur, such as a two-dimensional (2D) or three-dimensional (3D) approximation of the trochlea area. In an example, the computing system 140 can receive the landmarks illustrated in FIG. 3A above and can generate a bone model including a spline approximating a 2D profile between a distal most portion of the medial condyle and a canal entry point within the trochlea. In another example, the computing system 140 can receive the landmarks illustrated in FIG. 3B above and can generate a bone model including a 3D surface mesh approximation of the surface of the distal femur including the medial condyle and the trochlea. Bone models can also be generated using selected marks as inputs to a statistical bone atlas. Additionally, in some examples, medical imaging is available, and the bone model can be generated from the medical images and/or a combination of landmarks and medical imaging.

At 608, the technique 600 can continue with the computing device 140 simulating a resection. In an example, the resection is simulated using the bone model and the resection parameters. Finally, the technique 600 can complete at 610 with the computing system 140 generating a resection warning based on analysis of the simulated resection. In an example, generating the resection warning can include determining that the simulated resection resulted in notching the trochlea. In this example, notching the trochlea is defined as a partial knee distal femoral resection not being able to be completed without leaving a step/hard transition to the trochlea.

FIG. 7 is a flowchart illustrating a technique 700 for generating a trochlear notch warning in accordance with at least one example of this disclosure. In this example, the technique 700 can include operations such as accessing bone resection parameters at 702, analyzing resection parameters at 703, and generating a trochlear notch warning at 704. The technique 700 can begin at 702 with the computing system 140 accessing bone resection parameters. The bone resection parameters can include any of the various parameters discussed herein or generally known for resection of target bone.

At 703, the technique 700 can continue with the computing system 140 analyzing the resection parameters. In an example, analyzing the resection parameters can include comparing the resection parameters to safe ranges for each parameter. In some examples, analyzing the resection parameters can also include determining that a combination of parameters within the set of resection parameters triggers a warning condition. Analyzing the resection parameters can also optionally include processing the proposed resection parameters with a machine learning model training against a bone atlas covering a statistically significant range of bone sizes. In an example, the machine learning model (ML model) is trained by performing a plurality of simulated bone resections on a plurality of representative bone from the bone atlas. Optionally, performing the plurality of simulated bone resection can include each simulated bone resection of the plurality of bone resections using a unique set of bone cut parameters. Training the ML model can also include indexing each bone cut parameter through an allowable range to generate a large training set of simulated resections. Indexing each bone cut parameter through an allowable range can generate a complete set of allowable resections, which can then be used on each a bone model for each representative bone selected from the bone atlas to generate a simulated resection training set. Note, further details on training a machine learning model are discussed in reference to FIG. 5A, those details can all be implemented within the context of technique 700. Additionally, all of the details discussed in reference to using the machine learning model generated by FIG. 5A in making an inference, discussed in reference to FIG. 5B, can also be incorporated into technique 700.

At 704, the technique 700 can conclude with the computing system 140 generating a trochlear notch warning (or in other examples a different warning regarding a negative resection outcome). The warning can include identifying one or more out of range resection parameters. The warning can also include identifying one or more combinations of resection parameters responsible for the warning.

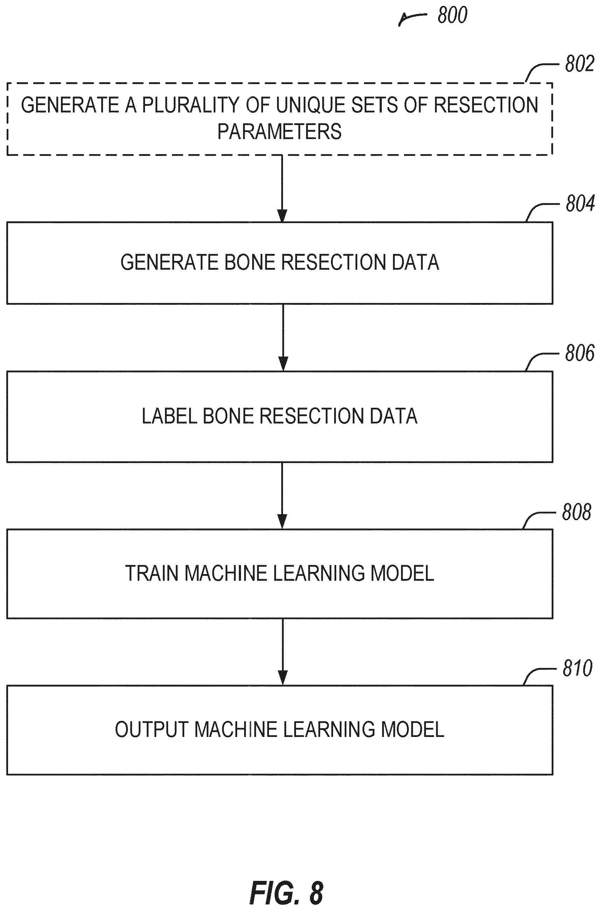

FIG. 8 is a flowchart illustrating a technique 800 for training a machine learning model to detect trochlear notching in accordance with at least one example of this disclosure. According to the invention, the technique 800 includes operations of generating bone resection data at 804, labeling bone resection data at 806, training a machine learning model at 808, and outputting a machine learning model at 810. Optionally, the technique 800 can also include generating a plurality of unique sets of resection parameters at 802. The machine learning model output by technique 800 can be used within the technique 700 for identifying/predicting negative resection outcomes, such as notching the trochlea.

At 802, the technique 800 can optionally begin with a computing device generating a plurality of unique sets of resection parameters. The plurality of unique sets of resection parameters can be generated by indexing each different resection parameter through an allowable range. The allowable ranges can reflect the options made available to a surgeon within a pre-operative or intra-operative surgical system, such as robotic surgical system 100, for a particular procedure. At 804, the technique 800 continues with the computing device generating bone resection data. The bone resection data is generated by performing simulated resections on a set of representative bone models using a representative set of resection parameters. The representative bone models can be selected from a statistical bone atlas. In some examples, the plurality of unique sets of resection parameters can be applied in simulated resections on the representative bone models. In certain examples, the plurality of unique sets of resection parameters can be used to perform simulated resections on the entire bone atlas to generate a large bone resection data set.

In some examples, the bone resection data is generated using landmark data applied to bone models from the bone atlas. In these examples, a plurality of landmarks are identified on each bone model to simulate digitizing landmarks on a target bone, such as discussed above. The landmarks are then used to generate a landmark based model of a portion of the bone model and a simulated resection is performed based on the landmark-based model. In an example, a second simulated resection is performed on the full bone model (e.g., the bone model from the bone atlas) and the result of a second simulated resection can be used to label the first simulated resection outcome (this labeling would be part of operation 806 discussed below).

At 806, the technique 800 continues with the computing device labeling the bone resection data generated at 804. In an example, the labeling is input into the computing device by a surgeon or trained health care provider. The labeling includes indicating a negative resection outcome, such as notching of the trochlear groove. As noted above, in certain example, the computing device can provide labeling input through comparison simulated resection outcomes with known good results. Labeling the bone resection data produces a training data set for use in training a machine learning model.

At 808, the technique 800 continues with the computing device training a machine learning model based on the training data. The machine learning model is trained to predict bone resection errors based on resection parameters from a pre-operative or intra-operative planning system. Training a machine learning model is discussed further above in reference to FIG. 5A. The technique 800 concludes at 810 with the computing device outputting the trained machine learning model.

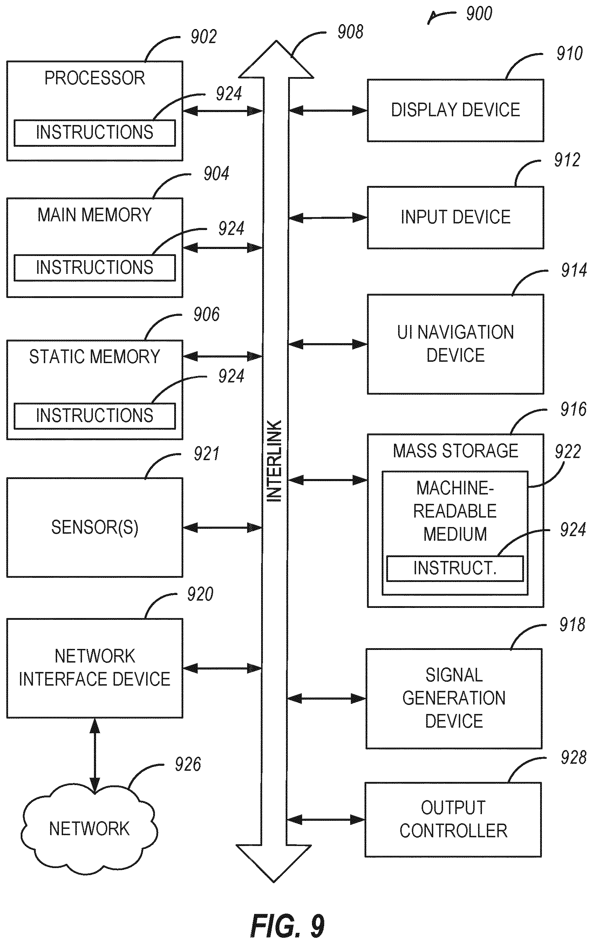

FIG. 9 illustrates a block diagram of an example machine 900 upon which any one or more of the techniques (processes) discussed herein may perform in accordance with some embodiments. In alternative embodiments, the machine 900 may operate as a standalone device and/or may be connected (e.g., networked) to other machines. In some examples, the machine 900 can be integrated into the robotic surgical system 100, such as depicted computing system 140. In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 900 may include a hardware processor 902 (e.g., a central processing unit (CPU), a graphics processing unit (GPU), a hardware processor core, or any combination thereof), a main memory 904 and a static memory 906, some or all of which may communicate with each other via an interlink (e.g., bus) 908. The machine 900 may further include a display unit 910, an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensor. The machine 900 may include an output controller 928, such as a serial (e.g., Universal Serial Bus (USB), parallel, or other wired or wireless (e.g., infrared (IR), near field communication (NFC), etc.) connection to communicate and/or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine readable medium 922 is illustrated as a single medium, the term "machine readable medium" may include a single medium or multiple media (e.g., a centralized or distributed database, and/or associated caches and servers) configured to store the one or more instructions 924. The term "machine readable medium" may include any medium that is capable of storing, encoding, or carrying instructions for execution by the machine 900 and that cause the machine 900 to perform any one or more of the techniques of the present disclosure, or that is capable of storing, encoding, or carrying data structures used by or associated with such instructions. Non-limiting machine-readable medium examples may include solid-state memories, and optical and magnetic media.

The instructions 924 may further be transmitted or received over a communications network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communications network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 900, and includes digital or analog communications signals or other intangible medium to facilitate communication of such software.

Method (technique) examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or nonvolatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A computer-implemented method for training a machine learning model (502) for use in predicting bone resection error, the method comprising:
generating (804) bone resection data from performing simulated bone resections on bone models within a bone atlas;
labeling (806) the bone resection data to generate training data;
training (808) the machine learning model (502), based at least in part on the training data, to predict bone resection errors based on bone cut parameters from a pre-operative planning system; and
outputting (810) the machine learning model (502).

2. The computer-implemented method of claim 1, wherein the generating (804) the bone resection data includes the performing simulated bone resections on each bone model within the bone atlas.

3. The computer-implemented method of claim 2, wherein each simulated bone resection of the simulated bone resections uses a unique set of bone cut parameters.

4. The computer-implemented method of claim 2, further comprising
generating (802) a plurality of unique sets of bone cut parameters by indexing through an allowable range for each bone cut parameter; and
wherein the performing the simulated bone resections includes applying the plurality of unique sets of bone cut parameters against at least a portion of the bone models within the bone atlas.

5. The computer-implemented method of claim 1, wherein the performing the simulated bone resections includes identifying a plurality of landmarks on each bone model of the bone models used in generating the bone resection data.

6. The computer-implemented method of claim 5, wherein the plurality of landmarks includes a plurality of landmarks between a distal most aspect of a femoral condyle and a canal entry point on a distal femur; and
wherein the performing the simulated bone resections includes generating a spline representative of a two-dimensional shape of a portion of the distal femur.

7. The computer-implemented method of claim 5, wherein the plurality of landmarks includes a plurality of landmarks between a j-curve of a femoral condyle and a canal entry point on a distal femur; and
wherein the performing the simulated bone resections includes generating a surface mesh representative of a three-dimensional shape of a portion of a distal femur.

8. The computer-implemented method of claim 1, wherein the labeling (806) the bone resection data includes identifying bone resections that result in notching of a trochlear groove.

## Patentansprüche

1. Computerimplementiertes Verfahren zum Trainieren eines Maschinenlernmodells (502) zur Verwendung beim Vorhersagen eines Knochenresektionsfehlers, wobei das Verfahren Folgendes umfasst:
Erzeugen (804) von Knochenresektionsdaten aus dem Durchführen von simulierten Knochenresektionen an Knochenmodellen innerhalb eines Knochenatlas;
Markieren (806) der Knochenresektionsdaten, um Trainingsdaten zu erzeugen;
Training (808) des Maschinenlernmodells (502), basierend zumindest teilweise auf den Trainingsdaten, um Knochenresektionsfehler basierend auf Knochenschnittparametern von einem präoperativen Planungssystem vorherzusagen; und
Ausgeben (810) des Maschinenlernmodells (502).

2. Computerimplementiertes Verfahren nach Anspruch 1, wobei das Erzeugen (804) der Knochenresektionsdaten das Durchführen von simulierten Knochenresektionen an jedem Knochenmodell innerhalb des Knochenatlas beinhaltet.

3. Computerimplementiertes Verfahren nach Anspruch 2, wobei jede simulierte Knochenresektion der simulierten Knochenresektionen einen einzigartigen Satz von Knochenschnittparametern verwendet.

4. Computerimplementiertes Verfahren nach Anspruch 2, ferner umfassend
Erzeugen (802) einer Vielzahl von einzigartigen Sätzen von Knochenschnittparametern durch Indizieren durch einen zulässigen Bereich für jeden Knochenschnittparameter; und
wobei das Durchführen der simulierten Knochenresektionen Anwenden der Vielzahl von einzigartigen Sätzen von Knochenschnittparametern gegen zumindest einen Teil der Knochenmodelle innerhalb des Knochenatlas beinhaltet.

5. Computerimplementiertes Verfahren nach Anspruch 1, wobei das Durchführen der simulierten Knochenresektionen Identifizieren einer Vielzahl von Orientierungspunkten an jedem Knochenmodell der Knochenmodelle, die beim Erzeugen der Knochenresektionsdaten verwendet werden, beinhaltet.

6. Computerimplementiertes Verfahren nach Anspruch 5, wobei die Vielzahl von Orientierungspunkten eine Vielzahl von Orientierungspunkten zwischen einem distalsten Aspekt einer Femurkondyle und einem Kanaleintrittspunkt an einem distalen Femur beinhaltet; und
wobei das Durchführen der simulierten Knochenresektionen Erzeugen eines Splines beinhaltet, der repräsentativ für eine zweidimensionale Form eines Teils des distalen Femurs ist.

7. Computerimplementiertes Verfahren nach Anspruch 5, wobei die Vielzahl von Orientierungspunkten eine Vielzahl von Orientierungspunkten zwischen einer j-Kurve einer Femurkondyle und einem Kanaleintrittspunkt an einem distalen Femur beinhaltet; und
wobei das Durchführen der simulierten Knochenresektionen Erzeugen eines Oberflächennetzes beinhaltet, das repräsentativ für eine dreidimensionale Form eines Teils eines distalen Femurs ist.

8. Computerimplementiertes Verfahren nach Anspruch 1, wobei das Markieren (806) der Knochenresektionsdaten Identifizieren von Knochenresektionen beinhaltet, die in Einkerben einer trochlearen Nut resultieren.

## Revendications

1. Procédé mis en œuvre par ordinateur permettant d'entraîner un modèle d'apprentissage automatique (502) destiné à être utilisé pour prédire une erreur de résection osseuse, le procédé comprenant :
la génération (804) de données de résection osseuse à partir de la réalisation de résections osseuses simulées sur des modèles osseux dans un os atlas ;
l'étiquetage (806) des données de résection osseuse pour générer des données d'entraînement ;
l'entraînement (808) du modèle d'apprentissage automatique (502), sur la base au moins en partie des données d'entraînement, pour prédire des erreurs de résection osseuse sur la base de paramètres de coupe osseuse à partir d'un système de planification préopératoire ; et
la sortie (810) du modèle d'apprentissage automatique (502).

2. Procédé mis en œuvre par ordinateur selon la revendication 1, ladite génération (804) de données de résection osseuse comprenant la réalisation de résections osseuses simulées sur chaque modèle osseux au sein de l'os atlas.

3. Procédé mis en œuvre par ordinateur selon la revendication 2, chaque résection osseuse simulée des résections osseuses simulées utilisant un ensemble unique de paramètres de coupe osseuse.

4. Procédé mis en œuvre par ordinateur selon la revendication 2, comprenant en outre
la génération (802) d'une pluralité d'ensembles uniques de paramètres de coupe osseuse par indexation dans une plage admissible pour chaque paramètre de coupe osseuse ; et
ladite réalisation des résections osseuses simulées comprenant l'application de la pluralité d'ensembles uniques de paramètres de coupe osseuse contre au moins une partie des modèles osseux au sein de l'os atlas.

5. Procédé mis en œuvre par ordinateur selon la revendication 1, ladite réalisation des résections osseuses simulées comprenant l'identification d'une pluralité de points de repère sur chaque modèle osseux des modèles osseux utilisés pour générer les données de résection osseuse.

6. Procédé mis en œuvre par ordinateur selon la revendication 5, ladite pluralité de points de repère comprenant une pluralité de points de repère entre un aspect le plus distal d'un condyle fémoral et un point d'entrée de canal sur un fémur distal ; et
ladite réalisation des résections osseuses simulées comprenant la génération d'une cannelure représentative d'une forme bidimensionnelle d'une partie du fémur distal.

7. Procédé mis en œuvre par ordinateur selon la revendication 5, ladite pluralité de points de repère comprenant une pluralité de points de repère entre une courbe en j d'un condyle fémoral et un point d'entrée de canal sur un fémur distal ; et
ladite réalisation des résections osseuses simulées comprenant la génération d'un maillage de surface représentatif d'une forme tridimensionnelle d'une partie d'un fémur distal.

8. Procédé mis en œuvre par ordinateur selon la revendication 1, ledit étiquetage (806) des données de résection osseuse comprenant l'identification de résections osseuses qui entraînent la formation d'incisures dans une rainure trochléaire.
